# EUROPEAN PATENT APPLICATION

(11) **EP 2 508 176 A1**
(43) Date of publication of application: **10.10.2012**
(21) Application number: 11161593.6
(22) Date of filing: 08.04.2011
(51) Int. Cl.: A61K 31/00, A61K 31/337, A61K 31/35, A61K 31/475, A61K 31/555, A61K 31/70, A61K 31/704, A61K 45/06, A61K 47/48, A61K 35/00, A61K 38/19

(54) **Novel combination treatment of cancer**

(71) Applicant: LIPOTARG GMBH, 4125 Riehen (CH)
(72) Inventor: Herrmann, Richard, 4125, Riehen (CH); Rochlitz, Christoph, 4125, Riehen (CH); Mamot, Christoph, 4054, Basel (CH)
(74) Representative: Cremer, Karsten

(57) **Abstract**

The present invention relates combination treatments in cancer therapy. Specifically, it provides the combination of EGFR-targeted immunoliposomes and colony-stimulating factors. The immunoliposomes preferably comprise an encapsulated cytostatic agent and an EGFR-targeting ligand such as an antibody, antibody fragment, or conjugate thereof. The colony-stimulating factors are selected from the group of granulocyte colony-stimulating factors, such as filgrastim, lenograstim, or pegfilgrastim. The immunoliposomes and the colony-stimulating factor may be administered separately or in a single composition.

## Description

### BACKGROUND

The present invention is in the area of cancer treatment. In particular, the invention relates to first-, second- and higher-line treatment of human patients suffering from cancer, particularly a cancer represented by a locally advanced or metastatic tumour. In a further aspect, the invention is related to composition which may be used in such treatment. Furthermore, the invention relates to combinations of active agents or compositions that are useful in the treatment of cancer.

The epidermal growth factor receptor (EGFR) is a tyrosine kinase receptor of the ErbB family that is expressed or activated in many epithelial tumours. Receptor activation leads to recruitment and phosphorylation of several downstream intracellular substrates, leading to mitogenic signalling and other tumour-promoting cellular activities. In human tumours, receptor overexpression correlates with a more aggressive clinical course (1, 2).

Several monoclonal antibodies (MAbs) directed to the ligand-binding extracellular domain of the receptor's tyrosine kinase are currently available for treatment or in late stage clinical development. Also under development are low-molecular weight inhibitors of the same target.

Among the known anti-EGFR MAbs, the one which is best characterized is the chimeric human:murine antibody cetuximab. Cetuximab is a potent inhibitor of the growth of cultured cancer cells that have an active autocrine EGFR loop. A series of phase I, phase II and phase III studies of cetuximab given alone or in combination either with chemotherapy or radiation were completed, leading to the market authorisation of cetuximab in most major countries. Cetuximab was found to be generally safe, but showed certain side effects including an acneiform skin rash in up to 40-70% of all treated patients. Anaphylactic or anaphylactoid reactions occurred in 2% of the patients. Non-neutralising human antibodies against chimeric antibodies were detected in 4% of the patients. The optimal biologic dose, as determined by saturation of antibody clearance, was found to be in the range of 200 to 400 mg/m² per week (3). Cetuximab is now considered part of standard therapy in patients with colorectal cancer and in head and neck tumours in many countries.

Doxorubicin is one of the most widely used anticancer drugs for the treatment of solid tumours and hematologic malignancies. It is active against a variety of cancer types, and is used extensively as a single agent and in combination chemotherapy regimens. In addition to its pivotal role in the treatment of breast cancer, doxorubicin has also demonstrated antitumour activity in ovarian, cervical, endometrial, gastric, bladder, and small-cell lung cancer, uterine sarcoma, acute lymphoblastic leukaemia, Hodgkin's and non-Hodgkin's lymphoma, multiple myeloma, and soft tissue and bone sarcomas. While doxorubicin displays an excellent antitumour activity profile, its use in clinical practice is limited by drug-associated toxicities, particularly myelosuppression and cardiotoxicity (see e.g., Principals and Practice of Oncology, DeVita, 6th edition).

Liposomal encapsulation of doxorubicin (e.g. MYOCET^{®}, Cephalon) was used to alter the tissue distribution and pharmacokinetics of the drug and to increase its therapeutic index. In particular, the liposomal form reduces the cardiotoxicity of doxorubicin.

Pegylated liposomal doxorubicin (DOXYL^{®}, Ortho Biotech Products LP, Bridgewater, NJ; CAELYX^{®}, Schering Plough, Kenilworth, NJ) is a new formulation of doxorubicin. Pegylation protects the liposomes from detection by the mononuclear phagocyte system and increases their circulation time, allowing for an increased exposure of the tumour cells to doxorubicin.

Pegylated liposomal doxorubicin has demonstrated efficacy as a single agent in patients with metastatic or recurrent breast cancer, with objective response rates ranging from 9% to 33% (4, 5). In comparison with conventional doxorubicin, pegylated liposomal doxorubicin has a similar efficacy profile and an improved safety profile, with a significantly reduced incidence of cardiotoxicity and significantly fewer cardiac events, as well as a reduced incidence of myelosuppression, mucositis, nausea, vomiting, and alopecia. On the other hand, pegylated liposomal doxorubicin is associated with palmar plantar erythrodysesthesia (PPE - hand-foot syndrome), a toxicity rarely or never seen with free doxorubicin.

In addition to its use in breast cancer, liposomal doxorubicin plays a well-established role in the treatment of Kaposi's sarcoma (6,7) and recurrent ovarian cancer (8): Moreover, it has been successfully used in patients with different types of lymphomas, multiple myeloma, soft tissue sarcoma, glioma, melanoma, mesothelioma, transitional cell carcinoma of the urothelial tract, in endometrial, pancreatic, gastric, small-cell and non-small-cell lung, hepatocellular, endometrial, renal cell, head and neck cancer, and cholangiocarcinoma (overview in (9)).

For conducting preclinical studies, anti-EGFR immunoliposomes were constructed by using Fab' fragments of the chimeric MAb cetuximab (C225; ERBITUX^{®}, ImClone Systems Corp., NY, USA; Merck KGaA, Darmstadt, Germany), which were covalently conjugated to phospholipid molecules which were incorporated within the liposome membrane. This approach was designed to provide maximal drug delivery to cancer cells via a receptor-targeted and internalizing drug carrier that is stable, non-immunogenic, and long-lived, with extended blood and tissue residence times and capable of delivering large payloads of diverse types of drugs to tumour cells. In parallel with MAb fragment optimization, conjugation methodology was also optimized. A new micellar incorporation method was developed involving 2-step conjugation of MAb fragments to preformed drug loaded liposomes (10). First, MAb fragments (Fab') were covalently conjugated to derivatized PEG-phosphatidyl-ethanolamine (MAL-PEG-DSPE) linkers in solution, resulting in immunoconjugates prone to spontaneous micelle formation. Next, the conjugates were incorporated into drug-preloaded liposomes by controlled heating, resulting in MAb fragments covalently conjugated to the termini of PEG chains and anchored to the liposome.

When Fab' of C225 was present at a moderate density on immunoliposomes (approx. 30 Fab' per liposome), these immunoliposomes displayed highly efficient binding and internalization in a panel of EGFR or EGFRvIII overexpressing cancer cell lines, as indicated by fluorescence microscopy and FACS (11). These included epidermoid cancer cells (A431), breast cancer cells (MDA-MB-468), malignant glioma cells (U87), and EGFRvIII stable transfectants NR6-M cells. In contrast, irrelevant immunoliposomes (anti-HER2) and control liposomes (no MAb) did not bind to or accumulate in A431, MDA-MB468, U87 or NR6-M cells. Also, anti-EGFR immunoliposomes did not detectably bind to, or accumulate in, non-EGFR-overexpressing cells (e.g. breast cancer cell lines SKBR-3 or MCF-7).

Under *in-vitro* conditions, quantitative studies of immunoliposome uptake, internalization, and intracellular drug delivery were performed using anti-EGFR immunoliposomes loaded with the pH-sensitive probe, HPTS. This method allows quantitative analysis of the kinetics of immunoliposome uptake at neutral pH (surface-bound) versus at acidic pH (endocytosis-associated) (12). In MDA-MB-468 cells, anti- EGFR immunoliposomes bound within 5 minutes, followed by intracellular accumulation beginning at 15 minutes and increasing up to 240 minutes. Total uptake of EGFR-targeted immunoliposomes in MDA-MB-468 cells when present at saturating concentrations was 1.70 fmol phospholipid/cell, which corresponds to an uptake of 13,000 liposomes/cell. Uptake of non-targeted liposomes in MDA-MB-468 cells was <300 liposomes/cell, indicating a more than 43-fold increase due to targeted delivery. Uptake of anti-EGFR immunoliposomes in non-EGFR overexpressing MCF-7 cells was 450 immunoliposomes/cell, indicating a 28-fold increased accumulation in EGFR-overexpressing MDA-MB468 cells.

In vivo, anti-EGFR immunoliposomes (ILs) showed extremely long circulation times as stable constructs in normal adult rats following a single i.v. dose, with pharmacokinetics that were indistinguishable from those of sterically stabilized stealth (long circulating) liposomes (13). Moreover, repeated administration revealed no increase in clearance, further confirming that immunoliposomes retain the long circulating and non-immunogenic characteristics of stealth liposomes. The potential therapeutic efficacy of anti-EGFR immunoliposomes loaded with a variety of anticancer agents (C225-ILs-dox) was evaluated in a series of tumour xenograft models (MDA-MB-468, U-87 and U-87vlll) (13).

The feasibility of the EGFR-targeted immunoliposomes for use in human therapy in a clinical setting has recently been demonstrated, using doxorubicin-loaded immunoliposomes whose surface was modified with Fab' fragments of C225 (WO 2009/040426 A1). In this study, no or very little drug-related toxicity was observed up to a concentration of 50 mg/m². Particularly, no skin toxicity, such as palmar plantar erythrodysesthesia, was found even after the highest doses that were administered. At the same time, clear signals of clinical efficacy were observed, even at the lowest dose used.

Drug resistance continues to be a major challenge in cancer treatment. In fact, within multi-line cancer treatment, one of the potential reasons for the observed lack of responsiveness is the development of a multi-drug resistance of tumour cells.

Intrinsic or acquired drug resistance occurs frequently in most cancers, and often involves simultaneous resistance to multiple agents (multidrug resistance, MDR). A number of mechanisms for drug resistance have been described. These include: overexpressed drug export pumps, such as P-glycoprotein (PGP) and multidrug-resistance protein (MRP); decreased drug uptake, such as altered folate carriers; inactivation of drugs, such as via glutathione-mediated reduction; overexpression of target enzymes, such as upregulated thymidylate synthase; altered drug targets, such as topoisomerase II; increased DNA repair capacity; reduced ability to undergo apoptosis; and others (reviewed in (30) and (31)). Among these mechanisms, the role of PGP in multidrug resistance has been one of the most intensively studied. PGP, encoded by the MDR1 gene, is a member of the ABC (ATP-Binding Cassette) transport protein family and is frequently over-expressed in the MDR phenotype. Other membrane-bound transporters capable of mediating drug efflux include multi-drug resistance protein MRP and other related proteins ((32), (33) and (34)). These proteins actively transport a variety of heterocyclic substrates, including cytotoxic drugs such as anthracyclines, vinca alkaloids, mitoxantrone, paclitaxel, and others out of the cell or into other cellular compartments ((32), (33) and (34)).

Specific inhibitors of these resistance mechanisms have been widely pursued as a means to restore drug sensitivity (reviewed in (35)). Although still actively under investigation, specific resistance inhibitors have yet to gain registration for clinical use. Progress towards therapeutic success has been hampered by such issues as inadequate specificity, both predictable and unforeseen toxicities, uncertainty about the true prevalence and contribution of the known resistance mechanisms, paucity of predictive assays to identify tumours dependent upon particular mechanisms, and multiplicity and redundancy of resistance mechanisms (35).

In spite of the progress achieved in recent years, it is still not possible to provide an effective and tolerable therapeutic treatment for every single cancer patient. In particular, patients that have developed a substantial drug resistance to one or more chemotherapeutic compounds considered as standard therapies may not respond to the available treatment options to such degree that complete tumour remission or and a substantial increase in survival time may be attained.

There is therefore a need for further improved cancer treatments. In particular, there is a need for providing further strategies for a safe therapeutic treatment of cancer patients, and especially of those patients who belong to the group of non-responders, i.e. who are not, or no longer, responsive to a conventional cancer chemotherapy. Moreover, there is a need for further strategies for overcoming intrinsic or acquired drug resistance in cancer therapy.

It is therefore an object of the present invention to provide an improved method for the treatment of cancer. In particular, it is an object to provide a treatment method which overcomes drug resistance, especially multi-drug resistance. In a further aspect, it is an object of the invention to provide a treatment method which is more effective than currently available methods in achieving tumour progression or increasing survival times. Moreover, it is an object to provide a treatment method which is associated with reduced toxicity compared with currently available methods. A yet further object is to provide treatment regimens which may be used within cancer treatment methods. Further objects of the invention will become clear on the basis of the description, the examples, and the patent claims.

### SUMMARY OF THE INVENTION

The present invention provides a combination of (a) an EGFR-targeted immunoliposome encapsulating a cytostatic agent and (b) a granulocyte colony-stimulating factor for use in the treatment of cancer.

In a second aspect, the invention provides a new method for treating a patient suffering from cancer. The method comprises the administration of (a) an EGFR-targeted immunoliposome encapsulating a cytostatic agent and (b) a granulocyte colony-stimulating factor for use in the treatment of cancer.

In a further aspect, the invention provides a pharmaceutical composition comprising an EGFR-targeted immunoliposome encapsulating a cytostatic agent for use in the treatment of cancer in patient, wherein the patient is further treated with a granulocyte colony-stimulating factor.

Moreover, the invention provides a pharmaceutical composition comprising a granulocyte colony-stimulating factor, for use in the treatment of cancer in patient, wherein the patient is further treated with EGFR-targeted immunoliposomes encapsulating a cytostatic agent.

In a particularly useful embodiment, the EGFR-targeted immunoliposomes are designed as long-circulating liposomes. Long-circulating characteristics may be achieved e.g. by incorporating PEGylated lipids within the liposomal membrane.

EGFR-targeted immunoliposomes may be prepared by linking EGFR-targeting moieties to the outer surface of liposomes. Such targeting moieties for EGFR may be represented by antibodies, antibody fragments, conjugates of antibodies or antibody fragments, or by small molecules having a high affinity for EGFR.

Examples of cytostatic agents which may be encapsulated in the EGFR-targeted immunoliposomes include alkylating agents, antimetabolites, antineoplastics, cytostatic plant alkaloids or terpenoids, and topoisomerase inhibitors. An example of a very suitable cytostatic agent is doxorubicin.

The granulocyte colony-stimulating factor used for carrying out the invention may be selected e.g. from filgrastim (rh-met-G-CSF), lenograstim (rh-G-CSF), and pegfilgrastim (PEG-rh-met-G-CSF).

### DETAILED DESCRIPTION OF THE INVENTION

In a first aspect, the present invention provides a combination of (a) an EGFR-targeted immunoliposome encapsulating a cytostatic agent and (b) a granulocyte colony-stimulating factor for use in the treatment of cancer.

Unexpectedly, it has been found by the inventors that by combining (a) EGFR-targeted immunoliposome loaded with a cytostatic agent and (b) a granulocyte colony-stimulating factor, the tolerability of the encapsulated cytostatic agent can be further improved, beyond the degree of tolerability improvement which is already achieved by the immunoliposomal encapsulation (over conventional liposomal encapsulation). The known effects of granulocyte colony-stimulating factors are related to their ability to accelerate recovery from neutropenia in cancer patients after chemotherapy with drug substances which cause myelosuppression. It could perhaps be expected that in cases in which dose-limiting myelosuppression occurs during therapy with a liposomal cytostatic agent, the co-administration of a granulocyte colony-stimulating factor would be beneficial and allow for the administration of higher amounts of the liposomal cytostatic agent. Surprisingly, however, also the severity of other toxic effects associated particularly with liposomal cytostatic agents such as liposomal doxorubicin which are not related to myelosuppression, especially hand-foot-syndrome (also known as chemotherapy- induced acral erythema (AE), palmoplantar erythema (PPE), or palmoplantar erythrodysesthesia syndrome) and mucositis, which may also limit the dose of the immunoliposomal cytostatic agent tolerated by patients, may be controlled by the combination therapy of the invention in such a way that the dose of the cytostatic agent may be further increased, beyond the dose increase which is already achievable by using EGRF-targeted immunoliposomes alone. Without wishing to be bound by theory, it is therefore not unlikely that the mechanism of toxicity reduction when carrying out the invention differs from the known mechanisms of action of granulocyte colony-stimulating factors.

In a related aspect, the invention provides a new method for treating a patient suffering from cancer. The method comprises the administration of (a) an EGFR-targeted immunoliposome encapsulating a cytostatic agent and (b) a granulocyte colony-stimulating factor for use in the treatment of cancer.

Further, the invention provides a pharmaceutical composition comprising an EGFR-targeted immunoliposome encapsulating a cytostatic agent for use in the treatment of cancer in patient, wherein the patient is further treated with a granulocyte colony-stimulating factor.

Moreover, the invention provides a pharmaceutical composition comprising a granulocyte colony-stimulating factor, for use in the treatment of cancer in patient, wherein the patient is further treated with EGFR-targeted immunoliposomes encapsulating a cytostatic agent.

As used herein, an immunoliposome is a liposome carrying on its surface a ligand having affinity to a target structure in an organism. Via such ligand, an immunoliposome is capable of binding selectively and specifically to the target. The target may be a molecular structure or moiety present in a cell or tissue. For example, the target may be the domain of a protein expressed by, and extending from the outer surface of the cell membrane of, certain tumour cells.

The ligand may be an antibody, an antibody fragment, a peptide, or a small molecule capable of recognising the target. The ligand may be directly incorporated in a liposome, or it may be conjugated to e.g. an anchoring moiety by which it can be incorporated into the liposomal membrane more readily.

A liposome is a small vesicle composed of amphiphilic lipids, optionally in combination with other excipients, which are capable of forming bilayer structures in an aqueous environment. The bilayers exhibit some similarity with biological membranes in that they are hydrophilic towards the inside and outside of the vesicles, whereas their lipophilic regions are sandwiched in-between these hydrophilic regions.

Liposomes typically have a size from less than about 100 nm to about 10 µm. Depending on their manufacture, they may comprise one or more concentric bilayers per vesicle. Small liposomes tend to be rather spherical, but larger vesicles may exist in various shapes.

Liposomes have been proposed as drug carriers. In fact, water-soluble compounds may be encapsulated within the aqueous inner compartment of a liposome. Very lipophilic molecules can also be associated with liposomes, in which they are usually incorporated within the lipophilic regions of the lipid bilayers.

Within the context of the invention, it is preferred that liposomes are used which are relatively small and thus appropriate for intravenous administration. Preferably, the average diameter of the liposomes as measured by photon correlation spectroscopy (PCS) is not larger than approx. 1,000 nm. In a further preferred embodiment, the average diameter is not more than about 400 nm. Further embodiments are directed to liposomes having an average particle size of not more than about 250 nm, or in the range from about 80 to about 250 nm, or in the range from about 100 to about 200 nm.

Moreover, in a further embodiment of the invention, the liposomes have a size suitable for extravasation into a solid tumour. This is particularly useful where the liposomes also include a surface coating of a hydrophilic polymer chain to extend the blood circulation lifetime of the liposomes. Liposomes remaining in circulation for longer periods of time, e.g., more than about 2-5 hours, are capable of extravasating into tumours and sites of infection, which exhibit compromised leaky vasculature or endothelial barriers. Such liposomes are typically between about 40-200 nm, more preferably between 50-150 nm, most preferably between 70-120 nm.

The amphiphilic lipids from which the liposomes are composed typically include at least one phospholipid. Phospholipids are amphiphilic lipids comprising a phosphate group, which is negatively charged and thus substantially hydrophilic. Phospholipids may be classified as glycerophospholipids (or phosphoglycerides, characterised by the presence of a glyceryl moiety) or phosphosphingolipids (or ceramides, such as sphingomyelin). Liposomes may contain native, semisynthetic and/or synthetic phospholipids.

Typically, liposomes comprise at least one glycerophospholipid (or phosphoglyceride). Such glycerophospholipids are in fact the most commonly used vesicle-forming lipids in liposomes. Commonly used glycerophospholipids include those which are derived from native lecithins, such as soy or egg lecithin, or from the (partial) hydration products thereof. Lecithins contain high amounts of phosphatidylcholines, but may also comprise smaller amounts of phosphoric acid, choline, fatty acids, glycerol, glycolipids, triglycerides, phosphatidylethanolamines, and phosphatidylinositol. Phosphatidylcholines are glycerophospholipid that comprise choline as a head group, in contrast to phosphatidylethanolamines and phosphatidylglycerols.

In phosphatidylcholines, two hydroxyl group of the glyceryl residue are linked via ester bonds to acyl groups, which are typically derived from medium to long chain fatty acids. Common acyl groups in phosphatidylcholines (but also in phosphatidylethanolamines and phosphatidylglycerols) used as constituents of liposomes include myristoyl, palmitoyl, stearoyl, and oleoyl groups.

Due to the negative charge of the phosphate group and the positive charge of the choline, phosphatidylcholines are always zwitterionic (sometimes also referred to as neutral). Phosphatidylethanolamines are also zwitterionic over large pH-ranges, but can exist as anions in basic environments. Phosphatidylglycerols are anionic.

In recent years, cationic liposomes have been found to be useful for certain purposes, such as for the encapsulation of genetic materials and for the transfection of cells therewith. Special cationic lipids have been designed and used for this purpose, including 1,2-dioleyloxy-3-(trimethylamino)propane (DOTAP), N-[1-(2,3-ditetradecyloxy)propyl]-N,N-dimethyl-N-hydroxyethylammonium bromide (DMRIE), N-[1-(2,3-dioleyloxy)propyl]-N,N-dimethyl-N-hydroxyethylammonium bromide (DORIE), N-[1-(2,3-dioleyloxy)propyl]-N,N,N-trimethylammonium chloride (DOTMA), 3-[N-(N',N'-dimethylaminoethane)carbamoylcholesterol (DC-Chol), and dimethyldioctadecylammonium (DDAB).

Besides one or more glycerophospholipids which serve as vesicle-forming lipids, liposomes may comprise one or more lipids which are themselves not capable of forming bilayers, but which modify or stabilise such bilayers. An example of such membrane-modifying lipid is cholesterol. In a particular embodiment, the immunoliposomes of the invention comprise at least one glycerophospholipid, in particular at least one phosphatidylcholine, and cholesterol.

Some or all of the vesicle-forming glycerophospholipids used in the immunoliposomes may be modified by derivatisation with a hydrophilic polymer. The purpose of such derivatisation is to modify the biological properties of the liposomes and, in particular, avoid their rapid uptake by the immune cells of the mononuclear phagocytic system (also referred to as the reticuloendothelial system, or RES), thus achieving longer circulation time in the blood after intravenous administration. As used herein, a long-circulating liposome is a liposome comprising an amount of amphiphilic lipids modified with a hydrophilic polymer which is effective to achieve a markedly prolonged circulation time. Preferably, the prolonged circulation time corresponds to an increase on the elimination half life of the liposomes in the bloodstream of at least about 1 hour, compared to similar liposomes without modified lipids. In further embodiments, the elimination half life is increased by at least 2 hours, 4 hours, 8 hours, 12 hours, or 24 hours, respectively.

Hydrophilic polymers suitable for derivatisation with a vesicle-forming lipid include polyvinylpyrrolidone, polyvinylmethylether, polymethyloxazoline, polyethyloxazoline, polyhydroxypropyloxazoline, polyhydroxypropylmethacrylamide, polymethacrylamide, polydimethylcrylamide, polyhydroxypropylmethacrylate, polyhydroxyethylacrylate, hydroxymethylcellulose, hydroxyethylcellulose, polyethylene glycol, polyethylene oxide, polyaspartamide and hydrophilic peptide sequences. The polymers may be employed as homopolymers or as block or random copolymers.

A preferred hydrophilic polymer chain is polyethylene glycol (PEG), preferably having a molecular weight between 200-20,000 Daltons, more preferably between 500-10,000 Daltons, still more preferably between 750-5000 Daltons. Methoxy- or ethoxy-capped analogues of PEG are also preferred as hydrophilic polymers, commercially available in a variety of molecular weights, such as from 120 to 20,000 Daltons.

The respective derivatised lipids, lipid-polymer conjugates, when incorporated within liposomal membranes, result in polymer chains extending from both the inner and outer surfaces of the liposomal lipid bilayers. The desired effect on the biological properties of the liposomes is typically achieved by including between 0.5-20 mole percent of the polymer-derivatised lipid with the remaining liposome forming components.

The preparation of vesicle-forming lipids derivatised with hydrophilic polymers has been described, for example in U.S. Pat. No. 5,395,619, in U.S. Pat. No. 5,013,556, in U.S. Pat. No. 5,631,018 and in WO 98/07409. It will be appreciated that the hydrophilic polymer may be stably coupled to the lipid, or coupled through an unstable linkage, which allows the coated liposomes to shed the coating of polymer chains as they circulate in the bloodstream or in response to a stimulus.

*Preparation of liposomes.* The immunoliposomes according to the present invention may be prepared by known methods for making liposomes having vesicle diameters that are suitable for parenteral administration. Perhaps the most simple method is the hydration of the vesicle constituents with an aqueous solution, which leads to the spontaneous formation of typically rather large vesicles, followed by the sizing of the liposomes using filtration or ultrasonication. Other methods which may be useful depending on the selection of the cytostatic agent which is to be encapsulated or with an eye on the desired properties of the liposomes include the polyol dilution method, the bubble method, the heating method, the ether (or solvent) injection method, the reverse-phase evaporation method, and the freeze-thaw method. For more details on these commonly known methods, reference is made to M. R. Mozafari, CELL. MOL. BIOL. LETT. Vol. 10. No. 4. 2005, pp. 711 ff. Moreover, the liposomes may be prepared by the methods disclosed in WO 2009/040426.

*Physical properties of liposomes.* Depending on the selected preparation method and manufacturing conditions, the resulting liposomes may be described as multilamellar vesicles (MLV), small unilamellar vesicles (SUV), or large unilamellar vesicles (LUV). MLVs differ from SUVs and LUVs in that MLVs have two or more lipid bilayers. Hence, MLVs appear useful in particular for being loaded with lipophilic drug substances which dissolve in, or associate with, the lipophilic regions of the vesicle membranes. In contrast, SUVs and LUVs are especially useful for the encapsulation of hydrophilic compounds within the aqueous compartment of the liposomes. Typically, MLVs have a diameter from about 200 nm up to several microns. SUVs typically range from about 80 nm to about 200-300 nm, whereas LUVs are normally understood to be larger than about 200-300 nm in average. Within the context of the invention, the diameters are understood as z-averages as measured with laser diffraction or photon correlation spectroscopy.

In one of the preferred embodiments, the liposomes of the invention are single- or multilamellar vesicles having an average diameter in the range from about 80 to about 500 nm, and in particular from about 100 to about 400 nm.

As mentioned, the desired particle sizes and the required degree of homogeneity in size may be achieved by various sizing methods, such as ultrasonication, high pressure homogenisation, and extrusion. The latter is typically performed by forcing an aqueous dispersion of the crude liposomes through one or more filters or membranes having defined pore sizes. For example, polycarbonate filters having pore diameters of about 400nm and 200 nm are commonly used for this purpose. The pore size of the last membrane used in the extrusion process roughly corresponds to the largest sizes of liposomes obtained thereby, particularly if the preparation is extruded two or more times through the same membrane.

*Cytostatic agent.* According to the invention, the EGRF-targeted immunoliposomes comprise an encapsulated cytostatic agent. As used herein, the term "encapsulated" refers to any form of association of the cytostatic agent with the liposome, whether by incorporation within the aqueous compartment or within the lipid compartment of the membrane(s).

A cytostatic agent is a pharmacologically active compound capable of inhibiting or suppressing cellular growth and multiplication. Depending on the mechanism of action and on the dose of the compound, it may also represent a cytotoxic agent. In particular, the cytostatic agent is a compound is capable of killing, or inhibiting the growth of, tumour cells.

The cytostatic agent may be selected, for example, from
(a) anthracyclines and analogs thereof, such as daunomycin, doxorubicin, idarubicin, epirubicin, valrubicin, aclacinomycin, mitoxantrone;
(b) antimetabolites, such as gemcitabine, cytosine arabinoside, cytarabine, vidarabine, thioguanine, pentostatin, cladribine, methotrexate, floxuridine, fluorouracil and other fluorinated pyrimidines, purines, or nucleosides;
(c) alkylating agents, such as nitrogen mustards, including cyclophosphamide, melphalan, chlorambucil, ifosfamide; nitrosoureas, including carmustine, lomustine, and streptozocin; alkyl sulfonates, including busulfan; thiotepa; platinum compounds, including cisplatin, carboplatin, oxaliplatin, nedaplatin, satraplatin, and triplatin tetranitrate; procarbazine; altretamine;
(d) plant alkaloids and terpenoids, such as vinca alkaloids, including vincristine, vinblastine, vinorelbine, and vindesine; taxanes, including taxol, paclitaxel, docetaxel; podophyllotoxin;
(e) topoisomerase inhibitors, such as amsacrine, etoposide, etoposide phosphate, teniposide and other derivatives of epipodophyllotoxins; irinotecan, topotecan and other camptothecins; and
(f) other antineoplastics, such as dactinomycin, bleomycin, mitomycin, etoposide, bleomycin, or plicamycin.

In one of the preferred embodiments, the encapsulated cytostatic agent is an anthracycline, in particular doxorubicin.

*Encapsulation of cytostatic agent.* The cytostatic agent may be encapsulated or incorporated within the liposomes by known techniques, either during the preparation of the liposomes, or subsequently, i.e. by loading preformed liposomes. For example, an aqueous solution of the cytostatic agent may be used to hydrate the vesicle-forming lipid composition, resulting in a portion of the active ingredient to be incorporated. Alternatively, the cytostatic agent may be incorporated into preformed liposomes by active transport mechanisms, e.g. in response to an ammonium, potassium or hydrogen ion concentration gradient (Mayer, 1986; Mayer 1989). Other methods, such as reverse-phase evaporation, may also be suitable. In one of the preferred embodiments, the incorporation of the cytostatic agent is performed as described in WO 2009/040426.

*EGFR-targeting ligand.* According to the invention, the immunoliposomes used in the combination treatment are further characterised in that they are EGFR-targeted. As used herein, EGFR-targeted means that the liposomes carry on their surface a ligand having affinity to EGFR. Via the ligand, the immunoliposomes are capable of binding selectively and specifically to EGFR. The ligand may be an antibody, an antibody fragment, a peptide, or a small molecule capable of recognising the target. The ligand may be directly incorporated in a liposome, or it may be conjugated to e.g. an anchoring moiety by which it can be incorporated into the liposomal membrane more readily.

As used herein, the EGF receptor or EGFR, also referred to as ErbB1 or HER1, means receptor protein which is a member of the class I family of receptor tyrosine kinases (RTKs), which includes EGFR (ErbB1 , HER1), HER2 (ErbB2), HER3 (ErbB3) and HER4 (ErbB4). As a target antigen, EGFR is a readily accessible cell surface receptor, which is overexpressed in many human solid tumours. Also included in this definition are mutants of EGFR, particularly Class III mutants such as EGFRvIII, which exhibits a deletion in exons 2-7 within the ECD, resulting in an in-frame deletion of 801 bp of the coding sequence and the generation of a novel glycine residue at the fusion junction.

Antibodies, as understood herein, include any type of antibody, including monoclonal, polyclonal, chimeric, single chain, bispecific, simianized, human and humanised antibodies. Preferably, the antibody or antibody fragment is an internalising antibody or fragment. As used herein, an internalizing antibody or fragment is an antibody or fragment that, upon binding to a receptor or other ligand on a cell surface, is transported into the cell, for example, into the cytoplasm, into a lysosome or another organelle.

A chimeric antibody is an antibody in which one or more regions of the antibody are from one species of animal and one or more regions of the antibody are from a different species of animal. A preferred chimeric antibody is one which includes regions from a primate immunoglobulin. A chimeric antibody for human clinical use is typically understood to have variable regions from a non-human animal, e.g. a rodent, with the constant regions from a human, in contrast, a humanised antibody uses CDRs from the non-human antibody with most or all of the variable framework regions from and ail the constant regions from a human immunoglobulin. A human chimeric antibody is typically understood to have the variable regions from a rodent. A typical human chimeric antibody has human heavy constant regions and human light chain constant regions with the variable regions of both the heavy and light coming from a rodent antibody, A chimeric antibody may include some changes to a native amino acid sequence of the human constant regions and the native rodent variable region sequence. Chimeric and humanised antibodies may be prepared by methods well known in the art including CDR grafting approaches (see, e.g., U.S. Patent Nos. 5,843,708; 6,180,370; 5,693,762; 5,585,089; 5,530,101), chain shuffling strategies (see e.g., U.S. Patent No. 5,565,332; (16), molecular modelling strategies (U.S. Patent No, 5,639,641}, and the like.

A humanised antibody refers to an engineered antibody which incorporates at least one humanised immunoglobulin or antibody chain or fragment thereof, particularly at least one humanised light or heavy chain. Said humanised immunoglobulin or antibody chain or fragment thereof, but particularly the at least one humanised light or heavy chain is derived from a non-human source, particularly a non-human antibody, typically of rodent origin. Said non-human contribution to the humanised antibody is typically provided in form of at least one CDR region which is interspersed among framework regions derived from one or more human immunoglobulins. In addition, framework support residues may be altered to preserve binding affinity.

The humanised antibody may further comprise at least one constant region or portion thereof, in the case of a light chain, and preferably three constant regions in the case of a heavy chain. Methods to obtain humanised antibodies are well known to those skilled in the art. (17). A humanised antibody may also be obtained by a novel genetic engineering approach that enables production of affinity-matured human-like polyclonal antibodies in large animals such as, for example, rabbits (http://www.rctech.com/bioventures/- therapeutic.php).

If an antibody fragment is used, such fragment may be selected from Fab, Fab', F(ab')₂, Fabc, Fv, single chains, single-chain antibodies, and the like. Preferably, the antibody or antibody fragment comprises at least one binding domain which specifically binds the EGR receptor on the surface of a tumour-derived cell.

Fragments can be obtained via chemical or enzymatic treatment of an intact or complete antibody or antibody chain. Fragments can also be obtained by recombinant means. Antigen-binding fragments refer to polypeptide fragments of an immunoglobulin or antibody that bind to the same antigen or compete with the intact antibody from which they are derived.

Antibody-binding fragments are produced by recombinant DNA techniques, or by enzymatic or chemical cleavage of intact immunoglobulins. Binding fragments include Fab, Fab', F(ab')2, Fabc, Fv, single chains, and single-chain antibodies.

These active fragments can be derived from a given antibody by a number of techniques. For example, purified monoclonal antibodies can be cleaved with an enzyme, such as pepsin, and subjected to HPLC gel filtration. The appropriate fraction containing Fab fragments can then be collected and concentrated by membrane filtration and the like. For further description of general techniques for the isolation of active fragments of antibodies, see for example (14); (15).

In one of the preferred embodiments, the EGFR-targeted immunoliposomes according to the present invention comprise a targeting ligand selected from the antibodies C225 (cetuximab), EMD72000, and antibody fragments obtained from these. Cetuximab or C225 is a chimeric human:murine antibody. It is known as a potent inhibitor of the growth of cultured cancer cells that have an active autocrine EGFR loop. In particular, the invention may be carried out using Fab' from C225. Methods for preparing Fab' from C225 are disclosed, for example, in WO 2009/040426.

The EGFR-targeting ligand may be attached to a liposome by methods known in the art. For example, a derivatized lipid containing an end-functionalized polyethylene glycol chain may be incorporated into liposomes. After liposome formation, the end-functionalized group can react with an antibody for antibody coupling to a liposome. There are a variety of techniques for attaching a selected hydrophilic polymer to a selected lipid and activating the free, unattached end of the polymer for reaction with a selected ligand, and in particular, the hydrophilic polymer polyethylene glycol (PEG) has been widely studied (18; 19; 20; 21 ; 22).

Typically, the PEG chains are functionalized to contain reactive groups suitable for coupling with, for example, sulfhydryl, amino, aldehyde or ketone groups (typically derived from mild oxidation of carbohydrate portions of an antibody) present in a wide variety of ligands. Examples of such PEG-terminal reactive groups include maleimide (for reaction with sulfhydryl groups), N-hydroxysuccinimide (NHS) or NHS-carbonate ester (for reaction with primary amines), hydrazide or hydrazine (for reaction with aldehydes or ketones), iodoacetyl (preferentially reactive with sulfhydryl groups) and dithiopyridine (thiol-reactive).

According to a further embodiment, the insertion method is used for incorporating a conjugate comprising a lipid residue and a targeting ligand into a liposome. In this method, preformed liposomes and are incubated with the targeting conjugate to achieve insertion of the targeting conjugate into the liposomal bilayers. In this approach, the preformed liposomes, which may already be loaded with the cytostatic agent, are prepared by a variety of techniques, such as those detailed in (23).

As already mentioned, multilamellar vesicles can be formed by simple lipid-film hydration techniques. In this procedure, a mixture of liposome-forming lipids of the type detailed above dissolved in a suitable organic solvent is evaporated in a vessel to form a thin film, which is then covered by an aqueous medium. The lipid film hydrates to form MLVs, typically with sizes between about 0.1 to 10 microns. The liposomes can include a vesicle-forming lipid derivatized with a hydrophilic polymer to form a hydrophilic surface coating on the liposomal surface. Addition of a lipid-polymer conjugate at the time of liposome formation is typically achieved by including between 0.5-20 mole percent of the polymer-derivatized lipid with the remaining vesicle-forming components. Subsequent sizing of the liposomes to achieve the preferred liposome diameters may be performed by homogenisation, extrusion or ultrasonication.

The lipid conjugate of the EGFR-targeting ligand is typically provided in the form of a micellar aqueous solution. For insertion of the lipid conjugate, the preformed liposomes and the - optionally micellar solution of the - conjugate are incubated under conditions allowing the association of the conjugate with the liposomes in such a way that at least some of the targeting ligand is incorporated and oriented outwardly from the liposome surface, and therefore available for interaction with its cognate receptor. It will be appreciated that the conditions effective to achieve such association or insertion are determined based on several variables, including, the desired rate of insertion, where a higher incubation temperature may achieve a faster rate of insertion, the temperature to which the ligand can be safely heated without affecting its activity, and to a lesser degree the phase transition temperature of the lipids and the lipid composition. It will also be appreciated that insertion can be varied by the presence of solvents, such as amphipathic solvents including polyethylene glycol, ethanol, or detergents.

*Immunoliposomal compositions.* The EGFR-targeted immunoliposomes may typically comprise at least about 50 wt.-% vesicle-forming lipids, a fraction of which (e.g. 0.5 to 30 wt.-%, relative to the total lipid content) may be derivatised with a hydrophilic polymer; up to about 50 wt.-% cholesterol; and about 0.0001 to about 10 wt.-% of the lipid conjugate of the EGFR-targeting ligand.

In a particular embodiment, the content of the EGFR-targeting ligand in the immunoliposome is in the range of between 1 µg to 150 µg per µmol phospholipid, particularly in the range of 5 µg to 100 µg of ligand per µmol phospholipid, particularly from 10 µg to 100 µg of ligand per µmol phospholipid, or from 20 µg to 50 µg, or from 30 µg to 40 µg of ligand per µmol of phospholipid, respectively.

In a further embodiment, the immunoliposomes comprise about 50 to about 75 wt.-% of a vesicle-forming phosphatidylcholine, such as purified and hydrated phosphatidylcholine from soy lecithin; about 10 to about 30 wt.-% of a phospholipid derivatised with a hydrophilic polymer, such as N-(carbonyl-methoxypolyethylene glycol 2000)-1,2-distearoyl-snglycero-3-phosphoethanolamine sodium salt (MPEG-DSPE); about 10 to about 30 wt.-% cholesterol, and up to 2 wt.-% (preferably about 10 to about 100 molecules per liposome) of the lipid conjugate of the EGFR-targeting ligand. According to a yet further embodiment, the EGFR-targeted immunoliposomes comprise about 60 wt.-% of a purified and hydrated phosphatidylcholine from soy lecithin; about 20 wt.-% N-(carbonyl-methoxypolyethylene glycol 2000)-1,2-distearoyl-sn-glycero-3-phosphoethanolamine sodium salt (MPEG-DSPE); about 20 wt.-% cholesterol, and about 10 to about 100 molecules per liposome of a conjugate obtained from coupling Fab' derived from C225 to Mal-PEG-DSPE; further comprising doxorubicin as an encapsulated cytostatic agent.

The EGFR-targeted immunoliposomes are preferably formulated as aqueous dispersions, with vesicle sizes suitable for parenteral administration, as described above, e.g. below about 200 nm. The compositions may comprise further pharmaceutical excipients, such as co-solvents, diluents, pH-adjusting agents including buffers, acids, and bases, surfactants, solubilising agents, preservatives, isotonising agents, absorption enhancers, stabilisers, antioxidants, and the like.

Solvents and co-solvents, other than water, should be restrictively used or avoided if possible as the composition is primarily intended for parenteral administration. If the incorporation of a solvent cannot be avoided, the excipient should be selected carefully and in consideration of its physiological acceptability. Potentially suitable co-solvents include, for example, ethanol, glycerol, propylene glycol or polyethylene glycol.

In order to provide a well tolerated composition, the preparation should be adjusted to a euhydric pH value. The term "euhydric" already implies that there may again be a divergence between pharmaceutical and physiological requirements so that a compromise has to be found which, for example, guarantees that the preparation is, from an economical point of view, just sufficiently stable during storage but, on the other hand, largely well tolerated. Preferably, the pH value lies in the slightly acidic to neutral region, i.e., between pH values of about 3.5 to 8.5. It is to be noted that deviations towards a weakly acidic environment can be tolerated better than shifts of the pH value into the alkaline region. A pH value in the range of about 4.5 to about 7.5 is particularly preferred.

For adjusting and, optionally, buffering the pH, physiologically acceptable acids, bases, salts, and combinations of these may be used. Suitable excipients for lowering the pH value or as acidic components of a buffer system are strong mineral acids, in particular, sulphuric acid and hydrochloric acid. Moreover, inorganic and organic acids of medium strength as well as acidic salts may be used, for example, phosphoric acid, citric acid, tartaric acid, succinic acid, fumaric acid, methionine, acidic hydrogen phosphates with sodium or potassium, lactic acid, glucuronic acid etc. However, sulphuric acid and hydrochloric acid are most preferred. Suitable for raising the pH value or as basic component for buffer system are, in particular, mineral bases such as sodium hydroxide or other alkali and alkaline earth hydroxides and oxides such as, in particular, magnesium hydroxide and calcium hydroxide, ammonium hydroxide and basic ammonium salts such as ammonium acetate or ammonium sulphate, as well as basic amino acids such as lysine or histidine, carbonates such as sodium or magnesium carbonate, sodium hydrogen carbonate, citrates such as sodium citrate etc.

Not primarily for physiological, but for pharmaceutical reasons, the incorporation of one or more excipients to achieve chemical stabilisation may be required. This may also depend on the specific cytostatic agent comprised in the liposomes. The most common degradation reactions of chemically defined active agents in aqueous preparations comprise, in particular, hydrolysis reactions, which may be limited, primarily, by optimal pH adjustment, as well as oxidation reactions. Examples for active agents which may be subject to oxidative attack are those agents that have olefinic, aldehyde, primary or secondary hydroxyl, ether, thioether, endiol, keto or amino groups. Therefore, in the case of such oxidation-sensitive active agents, the addition of an antioxidant, optionally in combination with a synergist, may be advisable or necessary.

Antioxidants are natural or synthetic substances which prevent or interrupt the oxidation of the active agents. These are primarily adjuvants which are oxidisable themselves or act as reducing agents, such as, for example, tocopherol acetate, reduced glutathione, catalase, peroxide dismutase. Synergistic substances are, for example, those which do not directly act as reactance in oxidation processes, but which counteract in oxidation by an indirect mechanism such as the complexation of metal ions which act catalytically in the oxidation, which is the case, for example, for EDTA derivatives (EDTA: ethylenediamine tetraacetic acid). Further suitable antioxidants are ascorbic acid, sodium ascorbate and other salts and esters of ascorbic acid (for example, ascorbyl palmitate), fumaric acid and its salts, malic acid and its salts, butyl hydroxy anisole, propyl gallate, as well as sulphites such as sodium metabisulfite. Apart from EDTA and its salts, citric acid and citrates, malic acid and its salts and maltol (3-hydroxy-2-methyl-4H-pyran-4-one) may also act as chelating agents. Optionally, the composition contains at least one antioxidant. In a further embodiment, it contains both an antioxidant and a chelating agent. The combination of a vitamin E derivative, in particular, vitamin E acetate, with an EDTA derivative, in particular, EDTA disodium salt, may be suitable.

In order to be well-tolerated, an injectable composition should, as far as possible, have a physiologic tonicity or osmolality. Thus, it may be desirable to incorporate an osmotically active excipient to control the osmolality of the aerosol. The content of this excipient (or excipients, if a combination of substances is used) should be selected to yield an osmolality of the aerosol which does not deviate too much from that of physiological fluids, i.e., from about 290 mOsmol/kg. However, in individual cases, a compromise has again to be found between the physical-chemical or pharmaceutical needs on one hand and the physiological requirements on the other hand. In general, an osmolality in the range of up to 600 mOsmol/kg may be acceptable, depending on the volume of the composition which is to be administered per dosing. In particular, an osmolality in the range of about 200 up to about 450 mOsmol/kg is preferred. In further embodiments, the osmolality is even closer to the physiological value, i.e. from about 220 to about 400 mOsmol/kg, or from about 250 to about 350 mOsmol/kg.

Suitable osmotically active excipients include, for example, innocuous mineral salts which react largely neutrally (unless such adjuvants are, at the same time to adjust or buffer the pH value), such as sodium, calcium or magnesium chloride, sulfate or phosphate. One of the particularly preferred members of these is sodium chloride. Further preferred excipients for this purpose are magnesium and calcium sulfate and chloride. As an alternative to the neutral mineral salts, physiologically safe organic compounds may be used as isotonising agent. Particularly suitable are water soluble substances with a relatively low molecular weight, for example, with a molecular weight of less than 300 or, better still, less than 200 and with a correspondingly high osmotic activity. Examples for such excipients are sugars and sugar alcohols, in particular, sucrose, glucose, trehalose, mannitol, sorbitol and isomaltol.

Among the optional excipients are preservatives, which may be considered less desirable for injectable compositions for safety and tolerability reasons. Therefore, in one of the embodiments, the composition is substantially free of preservatives. However, if the composition, or a medicament comprising the composition, is to be packaged in multiple unit dose containers, it may be necessary that a preservative is used in order to maintain sterility. Potentially useful preservatives include, for example, octadecyldimethylbenzyl ammonium chloride, hexamethonium chloride, benzalkonium chloride, benzethonium chloride, phenol, butyl or benzyl alcohol, alkyl parabens such as methyl or propyl paraben, catechol, resorcinol, cyclohexanol, 3-pentanol, and m-cresol.

Other optional stabilisers include amino acids such as glycine, glutamine, asparagine, histidine, arginine, or lysine, as well as peptides, albumin (e.g. human serum albumin), and surfactants, in particular non-ionic surfactants like polysorbates or poloxamers.

The concentration of liposomes in the compositions may be up to about 20 wt.-%. Due to certain restrictions with respect to e.g. the viscosity of the composition in the case of parenteral administration, the optimal liposome concentration may be substantially lower, such as from about 0.5 to about 10 wt.-%, or from about 1 to about 5 wt.-%.

The corresponding concentration of the cytostatic agent in the composition will depend on the selected compound, its dosing requirements, its aqueous solubility, and on the encapsulation efficiency. In the case of doxorubicin, for example, the concentration may be in the range from about 0.1 to about 10 mg/ml, and in particular from about 1 to about 5 mg/ml, such as about 2 mg/ml.

In view of the particularly preferred parenteral administration of the composition, it is preferred that the composition is sterile according to pharmacopoeial requirements. Generally speaking, it is preferred that the EGFR-targeted immunoliposome composition is formulated and processed such as to be adapted for parenteral administration.

*Administration of immunoliposomal compositions.* The immunoliposome composition may be administered in particular by parenteral or topical administration. Suitable parenteral routes include intravenous, intraarterial, subcutaneous, intramuscular, intratumoral, and intraperitoneal injection or infusion. In a preferred embodiment, the administration is performed by intravenous or intraarterial injection or infusion. Alternatively, topical, in particular mucosal (such as sublingual or buccal) administration may be considered.

The dose and the administration interval will largely depend on the selected cytostatic agent. In the case of doxorubicin-loaded immunoliposomes, for example, a single dose of about 10 to about 100 mg/m² body surface is presently preferred. It is noted that conventional liposomal doxorubicin compositions (e.g. Caelyx^{®} or Doxil^{®}) are dosed up to 50 mg/m², and usually lead to substantial adverse effects such as hand-foot-syndrome already at 40 mg/m² or lower. In contrast, it has been observed that doxorubicin-containing EGFR-targeted (C225 Fab') immunoliposomes according to the present invention can be dosed higher than 50 mg/m², usually without causing any significant hand-foot-syndrome or mucositis. Moreover, other adverse effects such as myolotoxicity and alopecia are absent or only very mild even at these high doses, in particular when the treatment with the immunoliposomes was combined with a granulocyte colony-stimulating factor, as outlined below. It is therefore one of the preferred embodiments that doxorubicin-containing EGFR-targeted immunoliposomes are administered at a single dose of about 50 to 100 mg/m², including doses of about 60 mg/m², about 70 mg/m², about 80 mg/m², and about 90 mg/m².

*Granulocyte colony-stimulating factors.* The granulocyte colony-stimulating factor used in the combination treatment according to the invention may be selected from human granulocyte colony-stimulating factors and their functional derivatives. Particularly preferred are filgrastim (rh-met-G-CSF; commercially available as, e.g., Neupogen^{®}), lenograstim (rh-G-CSF; commercially available as, e.g., Granocyte^{®}), and pegfilgrastim (PEG-rh-met-G-CSF; commercially available as, e.g., Neulasta^{®}).

All of the currently available granulocyte colony-stimulating factors and analogs are produced by recombinant DNA technology. Pegfilgrastim is a version of filgrastim which is modified with polyethylene glycol. The compounds are usually used to stimulate the proliferation and differentiation of granulocytes in certain cancer patients and bone marrow transplant recipients.

The fact that granulocyte colony-stimulating factors work synergistically with doxorubicin-loaded EGRF-targeted immunoliposomes and substantially decrease the incidence and/or severity of adverse effects such as hand-foot-syndrome and mucositis, allowing for a higher dose of the immunoliposomes to be administered, is surprising; it could not have been expected on the basis of the known mechanisms of action of granulocyte colony-stimulating factors.

For carrying out the invention, the granulocyte colony-stimulating factor is formulated and processed such as to be adapted for topical or, in particular, parenteral administration. The commercially available injectable formulations may be used, as well as any alternative compositions which are suitable for the intended route of administration.

*Administration of granulocyte colony-stimulating factors and combination treatment regimens.* In a particularly preferred embodiment, the granulocyte colony-stimulating factor is administered intravenously by bolus injection or short infusion, or subcutaneously. If filgrastim is selected, suitable daily doses may range from about 2 to about 150 µg/m², in particular from about 4 to about 50 µg/m², or from about 5 to about 20 µg/m². In the case of lenograstim, suitable daily doses may range from about 50 to about 1,000 µg/m², in particular from about 100 to about 500 µg/m², or from about 150 to about 300 µg/m², respectively. If the combination treatment is carried out with pegfilgrastim, the daily dose may be selected preferably in the range from about 1 to about 10 mg, for example 6 mg. In the case of pegfilgrastim which has a much longer half life than its non-pegylated analogs, it may be sufficient to administer only one single dose per treatment cycle in order to perform the combination therapy according to the invention. In contrast, filgrastim and lenograstim should preferably be administered once or twice per day over a period from about 2 to about 14 days, in particular at least about 3 days, or over a period from 4 to 12 days.

The EGRF-targeted immunoliposomes and the granulocyte colony-stimulating factor may be combined within one formulation and administered simultaneously. However, in a preferred embodiment, they are administered separately, using separate compositions. It is furthermore preferred that they are administered at different times. In particular, the granulocyte colony-stimulating factor may be administered after a lag time after the administration of the immunoliposomes. The lag time is preferably in the range from about 12 to about 72 hours, such as 24 hours or 48 hours.

In one of the preferred embodiments, doxorubicin-loaded EGRF-targeted (C225 Fab') immunoliposomes are administered at a doxorubicin dose of 50 mg/m² or more up to about 100 mg/m² followed by a dose of about 1 to 10 mg (e.g. 6 mg) of pegfilgrastim, wherein the pegfilgrastim is administered at least about 24 hours later than the immunoliposomes.

In a further preferred embodiment, the combination treatment is administered in more than one cycle, i.e. the administration of EGRF-targeted immunoliposomes and granulocyte colony-stimulating factor, whether sequential or in direct combination, is repeated at least once. For example, 2 to 10 cycles or even more, depending on the specific patient status and response, may be administered. The intervals, i.e. the time between the start of two subsequent cycles, are typically several weeks. The inventors have found that the increased tolerability which results from combining the EGRF-targeted immunoliposomes with the granulocyte colony-stimulating factor allows for rather short intervals, such as about 3 weeks or even less. Such short intervals lead to a high overall drug exposure and a further improved antitumour response, as has been observed in patients with various types of cancer.

As used herein, the expression "about 3 weeks" means intervals of 18 to 24 days.

*Indications.* The combination treatment according to the invention is useful in the management or therapy of various types of cancer, in particular locally advanced and/or metastatic tumours. In one of the preferred embodiments, the tumour exhibits EGFR-expressing or EGFR-overexpressing tumour cells. At the same time, it is noted that the detection of EGFR expression depends on factors such as the method which is used for this purpose, and it should not be concluded that the method of the invention cannot be used in cases in which EGFR expressing cells have not been determined.

EGFR-positive tumours, as used herein, include in particular tumours that contain at least 1 %, particularly at least 2%, 3%, 4% or 5%, particularly at least 10%, EGFR positive cells, detected e.g. by an immunohistochemistry test such as the FDA- approved EGFR pharmaDx kit ("DAKO" test; DAKO North America, Inc), the Zymed EGFR kit or the Ventana EGFR 3C6 antibody. In particular, said EGFR positive cells overexpress the EGFR antigen and/or mutants of EGFR, particularly Class III mutants like EGFRvIII.

The combination treatment may be used as first-line treatment, i.e. in patients who receive their first treatment of the cancer. In other embodiments, the invention is used for second- and higher line treatments, i.e. in patients who have already received one or more established treatments, but with no success or limited success. In one of the preferred embodiments, the combination treatment according to the invention is used in a patient who has already received at least two established therapies which have not resulted in a stable disease, partial response, or complete response with respect to the target lesion(s). In other preferred embodiments, the invention is used in a patient who has already received at least three, four, five, or more established therapies which have not resulted in a stable disease, partial response, or complete response.

According to a further preferred embodiment, the combination treatment of the invention is used in a patient who has developed a multi-drug resistance. The multi-drug resistance may for example include a resistance to one or more compounds selected from docetaxel, mitoxantrone, gemcitabine, capecitabine, oxaliplatin, interferon, sunitinib, sorafinib, cis- or carboplatin, doxorubicin, methotrexate, vincristin, vinorelbine, pemetrexed, gefitinib, etoposid, irinotecan, cyclophosphamide, topotecan, cyclophosphamide, paclitaxel, mitomycin, bevacizumab, trastuzumab, 5-FU, cetuximab, temozolomide, bevacizumab, procarbacine, CCNU, and BCNU.

Types of cancer for which the combination treatment may be used include breast cancer, colorectal cancer, pancreatic cancer, kidney cancer, prostate cancer, pancreatic cancer, urothelial cancer, oesophageal cancer, head and neck cancer, hepatocellular cancer, mesothelioma, Kaposi's sarcoma, recurrent ovarian cancer, soft tissue sarcoma, glioma, melanoma, small-cell and non-small-cell lung cancer, endometrial cancer, basal cell carcinoma, transitional cell carcinoma of the urothelial tract, cervical cancer, endometrial cancer, gastric cancer, bladder cancer, uterine sarcoma, multiple myeloma, soft tissue and bone sarcoma, and cholangiocarcinoma.

According to a further embodiment, the invention is used for the treatment of patients suffering from the following cancers and who have not responded to the following therapies:
(a) Prostate cancer with tumours progressed on hormonal treatment, docetaxel, and/or mitoxantrone;
(b) Pancreatic or gall bladder cancer with tumours progressed on gemcitabine, capecitabine, and/or oxaliplatin;
(c) Kidney cancer with tumours progressed on interferon, capecitabine, sunitinib, and/or sorafinib;
(d) Urothelial cancer with tumours progressed on cis- or carboplatin, gemcitabine, doxorubicin, methotrexate, and/or vincristine;
(e) Non-small cell lung cancer with tumours progressed on cis- or carboplatin, gemcitabine, vinorelbine, pemetrexed, docetaxel, and/or gefitinib;
(f) Small-cell lung cancer with tumours progressed on cis- or carboplatin, etoposide, irinotecan, doxorubicin, vincristine, cyclophosphamide, and/or topotecan;
(g) Mesothelioma with tumours progressed on cis- or carboplatin, gemcitabine, and/or pemetrexed;
(h) Breast cancer with tumours progressed on cis- or carboplatin, doxorubicin, vincristine, cyclophosphamide, paclitaxel, docetaxel, gemcitabine, vinorelbine, capecitabine, mitomycin, methotrexate, mitoxantrone, bevacizumab, and/or trastuzumab;
(i) Oesophageal cancer with tumours progressed on cis- or carboplatin, 5-FU, and/or docetaxel;
(k) Head & neck cancer with tumours progressed on cis- or carboplatin, 5-FU, docetaxel, and/or cetuximab;
(l) Brain tumours with tumours progressed on temozolomide, bevacizumab, irinotecan, vincristine, procarbacine, CCNU, and/or BCNU;
(m) Hepatocellular cancer with tumours progressed on sunitinib and/or sorafenib; and
(n) Colon and rectal cancer with tumours progressed on cetuximab, bevacizumab, oxaliplatin, irinotecan, capecitabine and/or 5-FU.

The invention is also illustrated by means of the following examples, which is however not intended to limit the scope of the invention.

### EXAMPLES

### Example 1: Preparation of EGFR-targeted immunoliposomes loaded with doxorubicin

Commercial samples of pegylated liposomal doxorubicin (Doxil^{®}) and C225 mAb, or cetuximab, (Erbitux^{®}; ImClone Systems, New York, NY) were obtained. Cetuximab was cleaved and reduced as described in (11). Fab' fragments were covalently conjugated to maleimide groups at the termini of PEG-DSPE chains (Mal-PEG-DSPE; Nektar, Huntsville, AL; ref. 8). Conjugation efficiencies were typically 30% to 50% for C225-Fab'. For incorporation into the liposomes (Doxil^{®}), the conjugates were co-incubated with the liposomal dispersion at 55°C for 30 minutes at a protein/liposome ratio of 30 µg Fab'/µmol phospholipid, resulting in incorporation efficiencies of 70% to 80%. All experiments were conducted under aseptic conditions. The immunoliposomes had an average diameter of approximately 100 nm.

### Example 2: Combination treatment

The EGFR-targeted immunoliposomes with encapsulated doxorubicin prepared according to example 1 were administered intravenously to a patient suffering from progressive head&neck cancer at a dose of 60 mg doxorubicin/m². After about 24 hours, 6 mg of pegfilgrastim (Neulasta^{®}) were injected subcutaneously.

The patient, who had previously received, but not responded to paclitaxel, 5-fluorouracil, hydroxyurea, showed no signs of hand-foot-syndrome, mucositis or alopecia after receiving six 4-week cycles of the combination treatment.

In other patients, good anti-tumour effectiveness and excellent tolerability with no signs of hand-foot-syndrome, mucositis or alopecia and were observed after several 3-week cycles of the combination treatment.

### REFERENCES

(1) Sridhar SS, Seymour L, Shepherd FA. inhibitors of epidermal-growth-factor receptors: a review of clinical research with a focus on non-small-cell lung cancer. Lancet Oncol 2003;4:397-40.
(2) Longley DB, McDermott U, Johnston PG. Predictive markers for colorectal cancer: current status and future prospects. Clin Colorectal Cancer 2003;2:223- 30.
(3) Baseiga J, Pfister D, Cooper MR, Cohen R, Burtness B, Bos M, et al. Phase I studies of anti-epidermal growth factor receptor chimeric antibody C225 alone and in combination with cisplatin. J Clin Oncol 2000; 18:904-14.
(4) O'Brien ME, Wigler N, Inbar M, Rosso R, Grischke E, Santoro A, et ai. Reduced cardiotoxicity and comparable efficacy in a phase II! trial of pegylated liposomal doxorubicin HCI (CAELYX/Doxil) versus conventional doxorubicin for first-line treatment of metastatic breast cancer. Ann Oncol 2004; 15:440-9.
(5) Keller AM, Mennel RG, Georgoulias VA, Nabholtz JM, Erazo A1 Lluch A, et ai. Randomized phase III trial of pegylated liposomal doxorubicin versus vinorelbine or mitomycin C plus vinblastine in women with taxane-refractory advanced breast cancer. J Clin Oncol 2004;22:3893-901,
(6) Northfelt DW, Dezube BJ, Thommes JA, Levine R, Von Roenn JH, Dosik GM, et al. Efficacy of pegylated-liposomal doxorubicin in the treatment of AIDS-related Kaposi's sarcoma after failure of standard chemotherapy. J Clin Oncoi 1997;15:653-9,
(7) Stewart S, Jablonowski H, Goebel FD, Arasteh K, Spittle M, Rios A, et al. Randomized comparative trial of pegylated liposomal doxorubicin versus bleomycin and vincristine in the treatment of AIDS-related Kaposi's sarcoma. International Pegylated Liposomal Doxorubicin Study Group. J Clin Oncol 1998;16:683-91.
(8) Gordon AN, Fleagle JT, Guthrie D1 Parkin DE, Gore ME, Lacave AJ. Recurrent epithelial ovarian carcinoma: a randomized phase III study of pegylated liposomal doxorubicin versus topotecan. J Clin Oncol 2001 ;19:3312-22.
(9) Orditura M, Quagiia F, Morgillo F, Martineili E Lieto E, De Rosa G, et al. Pegylated liposomal doxorubicin: pharmacologic and clinical evidence of potent antitumour activity with reduced anthracycline-induced cardiotoxicity (review). Oncol Rep 2004:12:549-56.
(10) Kirpotin DB, Hong K, Park JW, Shalaby R, Shao Y Zheng W, et al, Anti-HER2 immunoliposomes produced by spontaneous capture of an amphipathic poly(ethylene glycol)-anti-HER2 antibody conjugate into the liposome membrane. Proc. Amer. Assoc. Cancer Res. 2000; 41 :325.
(11) Mamot C, Drummond DC, Greiser U Hong K, Kirpotin DB, Marks JD, et al. Epidermal growth factor receptor (EGFR)-targeted immunoliposomes mediate specific and efficient drug delivery to EGFR- and EGFRviii-overexpressing tumour cells. Cancer Res 2003b; 63:3154-61,
(12) Kirpotin D, Park JW, Hong K, Zalipsky S, Li WL, Carter P, et al. Sterically stabilized anti-HER2 immunoliposomes: design and targeting to human breast cancer cells in vitro. Biochemistry 1997; 36: 66-75.
(13) Mamot C, Drummond DC, Nobie C, Kirpotin DB, Hong K, Park JW. EGFR/EGFRvIII targeted immunoliposomes significantly enhance the efficacy of multiple anticancer drugs in vivo. Cancer Res 2005:.
(14) Khaw, B. A. et ai. J. Nucl. Med. 23:1011-1019 (1982).
(15) Rousseaux et al. Methods Enzymology, 121 :663-69, Academic Press, 1986.
(16) Rader et al. Proc. Natl. Acad. Sci USA (1998) 95:8910-8915.
(17) Queen et al., Proc. Natl Acad Sci USA, 86:10029-10032 (1989), Hodgson et al., Biotechnology, 9:421 (1991).
(18) Alien, T. M., et al., Biochemica et Biophysica Acta, 1237:99-108 (1995)
(19) Zalipsky, S., Bioconjugate Chem., 4(4):296-299 (1993)
(20) Zalipsky, S., et al. FEBS Lett., 353:71-74 (1994)
(21) Zalipsky, S. et al., Bioconjugate Chemistry, 6(6):705-708 (1995)
(22) Zalipsky, S., in STEALTH LIPOSOMES (D. Lasic and F. Martin, Eds.) Chapter 9, CRC Press, Boca Raton, Fla. (1995)
(23) Szoka, F., Jr., et al., Ann. Rev. Biophys. Bioeng., 9:467 (1980) (24) Martin, F. J., in SPECIALIZED DRUG DELIVERY SYSTEMS - MANUFACTURING AND PRODUCTION TECHNOLOGY, P. Tyler Ed., Marcel Dekker, New York, pp. 267-316 (1990)
(25) Bartlett GK, J Bio Chem 1959, 234: 466-8
(26) Lasic DD et al, FEBS Lett 1992, 312: 255-8
(27) Haran G et al, Biochim Biophys Acta 1993, 1151 ; 201-15
(28) Neflis DF et at, Biotech Prog 2005, 21 : 221 -32
(29) Therasse P, Arbuck SG, Eisenhauer EA, Wanders J, Kaplan RS, Rubinstein L, et al. New guidelines to evaluate the response to treatment in solid tumours. European Organization for Research and Treatment of Cancer, National Cancer Institute of the United States, National Cancer Institute of Canada. J Natl Cancer Inst 2000;92:205-16.
(30) Keilen, J.A. (1994). The Phenomenon of multi drug resistance. In: Kellen JA (ed) Reversal of MultiDrug Resistance in Cancer, CRC Press, Boca Raton, pp. 1-21.
(31) Mickiey, L. & Fojo, AT. (1998), The MDR genes. In: Pinedo HM, Giaccone G (eds.) Drug Resistance in the Treatment of Cancer, Cambridge University Press, Cambridge, pp. 101-131.
(32) Cole, S.P. & Deeley, R.G. (1998). Multidrug resistance mediated by the ATP- binding cassette transporter protein MRP. Bioessays, 20, 931-40.
(33) Juranka, P.P., Zastawny, R.L. & Ling, V. (1989). P-glycoprotein: multidrug-resistance and a superfamily of membrane- associated transport proteins. Faseb J, 3, 2583-92.
(34) Renes, J., de Vries, E.G., Jansen, P.L. & Muller, M. (2000). The (patho)physiological functions of the MRP family. Drug Resist Updates, 3, 289-302.
(35) Leonard, G.D., Polgar, 0. & Bates, S. E. (2002). ABC transporters and inhibitors: new targets, new agents. Curr Opin investig Drugs, 3, 1652-9.
(36) Fan, Z., Masui, H., Alias, [iota]. & Mendelsohn, J. (1993), Blockade of epidermal growth factor receptor function by bivalent and monovalent fragments of 225 anti-epidermal growth factor receptor monoclonal antibodies. Cancer Res, 53, 4322- 8.
(37) Bier, H., Hoffmann, T., Hauser, U., Wink, M., Ochier, M., Kovar, A., Muser, M. & Knecht, R. (2001). Clinical trial with escalating doses of the antiepidermal growth factor receptor humanised monoclonal antibody EMD 72 000 in patients with advanced squameous ceil carcinoma of the larynx and hypopharynx. Cancer Chemother Pharmacol, 47, 519-24.
(38) Claassen, E. (1992). Post-formation fluorescent labelling of liposomal membranes. In vivo detection, localisation and kinetics, J Immunol Methods, 147, 231-40
(39) Litzinger, D.C., Butting, A.M., van Rooijen, N. & Huang, L. (1994). Effect of liposome size on the circulation time and intraorgan distribution of amphipathic poly(ethylene glycol)-containing liposomes. Biochim Biophys Acta, 1190, 99-107
(40) Park, J.W., Hong, K., Carter, P., Asgari, H., Guo, L.Y., Keller, G.A., Wirth, C, Shalaby, R., Kotts, C, Wood, W.I. & et al. (1995). Development of anti-p185HER2 immunoliposomes for cancer therapy. Proc Natl Acad Sci U S A, 92, 1327-31.
(41) Scudiero, D.A., Shoemaker, R.H., Paull, K.D., Monks, A., Tierney, S., Nofziger, T.H., Currens, M.J., Seniff, D. & Boyd, Ivl.R. (1988). Evaluation of a soluble tetrazoiium/formazan assay for cell growth and drug sensitivity in culture using human and other tumour cell lines. Cancer Res, 48, 4827-33.
(42) O'Brien, P. C. (1984). Procedures for comparing samples with multiple endpoints. Biometrics, 40, 1079-87.
   U.S. Patent Nos. 5,843,708; 6,180,370; 5,693,762; 5,585,089; 5,530,101, 5,565,332; 5,639,641, 5,395,619, 5,013,556, 5,631,018, and WO 98/07409

## Claims

1. A combination of
(a) an EGFR-targeted immunoliposome encapsulating a cytostatic agent and
(b) a granulocyte colony-stimulating factor
for use in the treatment of cancer.

2. A pharmaceutical composition comprising an EGFR-targeted immunoliposome encapsulating a cytostatic agent for use in the treatment of cancer in patient, wherein the patient is further treated with a granulocyte colony-stimulating factor.

3. The composition of claim 2, wherein the cytostatic agent is selected from
(a) anthracyclins and analogs thereof, such as daunomycin, doxorubicin, idarubicin, epirubicin, valrubicin, aclacinomycin, mitoxantrone;
(b) antimetabolites, such as gemcitabine, cytosine arabinoside, cytarabine, vidarabine, thioguanine, pentostatin, cladribine, methotrexate, floxuridine, fluorouracil and other fluorinated pyrimidines, purines, or nucleosides;
(c) alkylating agents, such as nitrogen mustards, including cyclophosphamide, melphalan, chlorambucil, ifosfamide; nitrosoureas, including carmustine, lomustine, and streptozocin; alkyl sulfonates, including busulfan; thiotepa; platinum compounds, including cisplatin, carboplatin, oxaliplatin, nedaplatin, satraplatin, and triplatin tetranitrate; procarbazine; altretamine;
(d) plant alkaloids and terpenoids, such as vinca alkaloids, including vincristine, vinblastine, vinorelbine, and vindesine; taxanes, including taxol, paclitaxel, docetaxel; podophyllotoxin;
(e) topoisomerase inhibitors, such as amsacrine, etoposide, etoposide phosphate, teniposide and other derivatives of epipodophyllotoxins; irinotecan, topotecan and other camptothecins; and
(f) other antineoplastics, such as dactinomycin, bleomycin, mitomycin, etoposide, bleomycin, or plicamycin.

4. The composition of claim 2 or 3, wherein the cancer includes EGFR-expressing tumour cells.

5. The composition of any of claims 2 to 4, wherein the cancer exhibits a multi-drug resistance.

6. The composition of any of claims 2 to 5, wherein the immunoliposome comprises an EGFR-targeted antibody, antibody fragment, conjugate of an antibody, or conjugate of an antibody fragment.

7. The composition of claim 6, wherein the immunoliposome comprises a conjugate of a fragment of cetuximab or matuzumab.

8. The composition of any of claims 2 to 7, wherein the immunoliposome is a long-circulating liposome.

9. The composition of any of claims 2 to 8, wherein the granulocyte colony-stimulating factor is selected from filgrastim (rh-met-G-CSF), lenograstim (rh-G-CSF), and pegfilgrastim (PEG-rh-met-G-CSF).

10. The composition of any of claims 2 to 9, wherein the granulocyte colony-stimulating factor is administered 12 to 72 hours after the administration of the EGFR-targeted immunoliposome.

11. The composition of any of claims 2 to 10, wherein the EGFR-targeted immunoliposome is administered at a single dose which comprises a higher amount of encapsulated cytostatic agent than administered in conventional therapy with said agent.

12. The composition of any of claims 2 to 11, wherein the EGFR-targeted immunoliposome and the granulocyte colony-stimulating factor are administered in at least two treatment cycles **characterised by** an interval of less than 4 weeks, in particular about 3 weeks.

13. A method for treating a patient suffering from cancer, comprising the administration of
(a) an EGFR-targeted immunoliposome encapsulating a cytostatic agent and
(b) a granulocyte colony-stimulating factor for use in the treatment of cancer.

14. The method of claim 13, wherein the cancer is a locally advanced or metastatic cancer exhibiting EGFR-expressing cancer cells and optionally selected from breast cancer, colorectal cancer, pancreatic cancer, kidney cancer, prostate cancer, pancreatic cancer, urothelial cancer, oesophageal cancer, head and neck cancer, hepatocellular cancer, mesothelioma, Kaposi's sarcoma, recurrent ovarian cancer, soft tissue sarcoma, glioma, melanoma, small-cell and non-small-cell lung cancer, endometrial cancer, basal cell carcinoma, transitional cell carcinoma of the urothelial tract, cervical cancer, endometrial cancer, gastric cancer, bladder cancer, uterine sarcoma, multiple myeloma, soft tissue and bone sarcoma, and cholangiocarcinoma.

15. The method of any of claims 13 or 14, wherein the patient, prior to receiving the treatment according to said method, did not exhibit dose-limiting myelosuppression under treatment with an EGFR-targeted immunoliposome encapsulating a cytostatic agent without co-administration of a granulocyte colony-stimulating factor.
